# EUROPEAN PATENT APPLICATION

(11) **EP 4 685 810 A1**
(43) Date of publication of application: **28.01.2026**
(21) Application number: 24190683.3
(22) Date of filing: 24.07.2024
(51) Int. Cl.: G16H 40/40

(54) **IOMT ENABLED ENDOSCOPE STATUS DISPLAY DEVICE**

(71) Applicant: Olympus Winter&Ibe GmbH, 22045 Hamburg (DE)
(72) Inventor: MÜCKNER, Andreas, 22045 Hamburg (DE)
(74) Representative: Weickmann & Weickmann PartmbB

(57) **Abstract**

An endoscope management device 10 comprises: a display device 12, an ID reader 14, a microprocessor 16, a countdown-timer 18, and a communication interface 20 for wirelessly connecting the endoscope management device 10 to an external communication network 21, wherein the endoscope management device 10 is configured to: detect an endoscope ID code of a freshly pre-cleaned endoscope 22 by means of the ID reader 14; start the countdown-timer 18 indicating a remaining time until a predetermined time interval starting with the detection of the endoscope ID code ends; display current endoscope information on the display device 12, and make the current endoscope information available in the communication network 21; the current endoscope information comprising: a name and/or serial number associated with the detected endoscope ID, and the current remaining time indicated by the countdown-timer 18.

## Description

The present application relates to an endoscope management device (EMD) and an endoscope management method.

Currently, several million endoscopic procedures are performed worldwide each year for a wide range of diagnostic and therapeutic purposes.

Endoscopic instruments are introduced into the body of a patient and can come into contact with a multitude of potentially harmful substances and microorganisms. Infections caused by cross-contamination due to improperly handled endoscopic instruments thus pose a serious health risk.

Therefore, it is extremely important to correctly follow established guidelines for reprocessing the respective endoscopic instrument after each use, the reprocessing procedure comprising e.g. steps of cleaning, disinfecting, checking and/or storing the instrument after each use.

In order to comply with legal and/or hospital specific requirements, each step of the reprocessing procedure has to be carefully documented. Conventional paper-based methods of documentation are time-consuming and prone to error. Therefore, digital information management and/or documentation systems such as Hytrack ^{™} have been established for managing endoscope information related to all endoscopes of a given endoscope pool and in particular for digitally keeping track of all the different steps of the reprocessing procedure, wherein the information management system is connected or connectable to a communication network, linking the endoscope pool with all connected devices and users.

After any usage of an endoscope in the procedure room, the endoscope is first manually pre-cleaned by a healthcare professional and is placed in a container for transport to a manual cleaning station where a more thorough manual cleaning procedure is performed. Afterwards, the endoscope is disinfected in an automated endoscope treatment and disinfection device (ETD device).

The focus of the present invention is however the first phase of the reprocessing procedure up to the arrival of the pre-cleaned endoscope at the manual cleaning station.

The time interval between the end of the manual pre-cleaning step on the one hand and the beginning of the first cleaning process in the manual cleaning station on the other hand should be lower than a predetermined time interval of currently e.g. 30 minutes. If this time interval is exceeded, additional measures are required before the instrument may be processed any further.

Against this background, the object of the present invention is to better support the healthcare professionals to keep the processing time low in the first phase of the reprocessing procedure.

In order to solve this object, the present invention proposes an endoscope management device (EMD) with the features of claim 1, the device comprising: a display device, an ID reader, a microprocessor, a countdown-timer, optionally integrated into the microprocessor, and a communication interface for wirelessly connecting the endoscope management device to an external communication network.

The term "countdown-timer" is to be understood as a functional component and does not necessarily refer to a physically separate element or a hardware unit. It can e.g. be implemented using a real-time clock provided in the EMD, in particular integrated into the microprocessor and e.g. using suitable software. Using a real-time clock makes it possible to provide accurate timestamps which could be used to coordinate other activities involving the EMD. Furthermore, it helps to hold the accurate time of an event and could support energy saving, making it possible to shut down the microcontroller and wake it up after a fixed period of time or at a particular later time.

The ID reader can be an NFC reader, an RFID reader, a barcode reader or any other suitable mechanism for reading an ID code.

According to the invention, the endoscope management device is configured to: detect an endoscope ID code of a freshly pre-cleaned endoscope by means of the ID reader; start the countdown-timer indicating or outputting a remaining time until a predetermined time interval ends or a predetermined target time is reached; display current endoscope information on the display device, and make the current endoscope information available in the communication network by means of the communication interface; the current endoscope information comprising: a name and/or serial number associated with the detected endoscope ID code, and the current remaining time as indicated/output by the countdown-timer.

The start of the countdown-timer can be triggered in different manners as described in more detail below with respect to the endoscope management method according to the invention.

Optionally, an ID code or any other information for identifying the endoscope management device can be made available in the communication network by means of the communication interface together with the current endoscope information so that it is possible to track which endoscope management device is currently associated with a given endoscope.

The display device, which is usually an electronic display device, can be implemented in any suitable form. In particular, it can be implemented as or can comprise an e-paper tag, an LCD screen, a seven-segment display, an LED or LED arrangement or any combination thereof.

The current remaining time does not necessarily have to be indicated in numeral or text form but could also be indicated e.g. by the color and/or blinking frequency of a light or light arrangement.

It is noted that the term e-paper or electronic paper refers to any electronic display device that does not emit light but reflects ambient light, like paper, and it is not limited to any particular technique for achieving this effect.

E-paper tags are known and widely used, for example as price tags in supermarkets. Wireless connectivity in these known tags is used for uploading new information on the e-paper tag, such as the name and current price of the product associated with the tag.

The present invention proposes to use the display device involving for example e-paper tag technology in order to better support the healthcare staff in the first phase of the reprocessing procedure for endoscopes. In particular, the display device as described herein makes it possible to quickly determine, ideally with a single glance at the display device, how much time is left before the particular endoscope has to reach e.g. the manual cleaning station even during the transport and to make this information also available in the communication network to other users or devices linked to the network. This allows to inform users also in other places about the status of the endoscope, e.g. on a dashboard on the floor of the endoscope department of the hospital.

The display device, the microprocessor, the countdown-timer and the communication interface as well as further components such as a battery and, if desired, additional memory, can be accommodated in a single housing preferably adapted to the hygiene requirements for medical devices. As the size of the housing is mainly determined by the desired screen size for the display device, the entire device can also be described as an "loMT enabled endoscope status display device" (loMT ESDD), the abbreviation IoMT referring to the term "Internet of Medical Things"

This loMT enabled endoscope status display device according to the invention as described herein can be attached to or integrated with a transport container configured to temporarily store the pre-cleaned endoscope e.g. on its way to the manual cleaning station, and protection is also claimed for such a transport container with an loMT enabled endoscope status display device.

According to the invention as described above, the ID reader is configured to read the endoscope ID code of a freshly pre-cleaned endoscope. Furthermore, the ID reader can also be configured to read an operator ID code of the healthcare professional currently in charge of the reprocessing procedure, e.g. from a staff identification tag, and information related to the operator ID code can also be displayed on the display device and/or made available to the communication network via the communication interface of the endoscope management device.

Preferably, the detection of the endoscope ID code and optionally also of the operator ID code or any other ID code as described below does not require any active involvement of the operator but the ID reader is configured and positioned in such a manner that the detection automatically happens during the precleaning process or when the endoscope is put into a transport container after the precleaning, e.g. by attaching the EMD and therefore also the ID reader to the transport container or integrating it therewith.

In order to automatically detect what currently happens to the endoscope and thus to reduce the documentation effort for the healthcare professional in charge of reprocessing, the device can furthermore comprise a motion sensor. The detected motion data are collected and optionally analyzed by the microprocessor, and the detected motion data and/or information derived therefrom can be made available in the communication network and/or can be shown on the display device.

The motion sensor can for example be or can comprise an accelerometer or a gyroscope.

Based on the motion data, the microprocessor is preferably configured to recognize at least one and preferably any one of the following events:
- the endoscope is placed inside a container to which the endoscope management device is attached,
- the endoscope is removed from the container,
- the container accommodating the endoscope is transported,
- the container without the endoscope is transported.

According to a preferred embodiment, the microprocessor can be configured to learn to recognize at least one of and preferably all of the events listed above based on the motion data detected by the motion sensor. In particular, suitable machine learning algorithms (tiny ML) enabling the microprocessor to learn to recognize these events can be installed on the microprocessor of the endoscope management device.

In any case, the device can be configured to make information related to the recognized event available in the communication network for further distribution and/or to display information related to the recognized event on the display device.

It might be advisable to equip the endoscope management device also with some additional localization means using e.g. Bluetooth or UWB, in order to make it possible to quickly determine the current location of the device, wherein the device is configured to make the determined current location of the device available in the communication network. Preferably, the additional localization means is also accommodated in the housing of the display device. This allows to quickly track and find a particular endoscope if needed and it allows to create an overview of all current locations of all endoscopes of the pool, facilitating the management of resources.

Furthermore, the endoscope management device can comprise acoustic and/or optical warning means such as a flashing LED or a buzzer, which also can help to quickly find the device. The warning means is also preferably accommodated within or attached to the housing of the display device.

In particular, the warning means can be adapted to emit a warning signal if the current remaining time indicated by the countdown timer falls below a predetermined threshold, the warning signal underlining an urgent need for further processing. It is also possible to use an LED which changes color depending on the remaining time until the endoscope has to reach the next station of the reprocessing procedure.

As mentioned above, protection is also claimed for a transport container for an endoscope, the container comprising an endoscope management device (loMT enabled ESDD) as described above that is attached to and/or integrated with the container. The container is preferably at least partially transparent and complies with the requirements for medicinal devices. In particular, it can be provided that the EMD, when being attached to the container, cannot change its position relative to the container which can be important in particular if the EMD comprises a motion sensor.

According to a particular embodiment of the invention, the EMD can be removably or exchangeably attached to the transport container, in particular if the EMD is not configured to withstand the conditions regarding e.g. temperature and/or humidity that might occur later in the treatment e.g. in the cleaning or disinfection process and if the endoscope is to be treated while being accommodated in the transport container.

As an alternative, it is also possible to integrate parts of the EMD with the transport container and to provide that only those components of the EMD are removably attached to the rest of the EMD and therefore to the container, which are especially sensitive. For example an e-paper tag forming the display device could be removably provided on the rest of the EMD device and/or on the container integrated with the rest of the EMD device.

In case the EMD can be removed from the transport container, it is preferred that also the transport container has a unique container ID code to be preferably automatically read by the ID reader of the EMD device when the EMD is attached to the transport container, so that it can be identified which transport container, which EMD and which endoscope are temporarily associated with each other, and also this information can be made available in the network.

Protection is also claimed for an endoscope management system for a pool of endoscopes, each endoscope having a unique ID code, the system comprising a plurality of endoscope management devices and/or transport containers as described above, the endoscope management devices all being connected or connectable to the same communication network. Furthermore, the system comprises an information management system, the information management system being configured to manage the current endoscope information made available in the communication network by all the endoscope management devices.

In this manner, the management of the endoscope pool e.g. of a hospital can be facilitated. The system can also comprise the pool of endoscopes.

Preferably, the system can also comprise a dashboard configured to display the current endoscope information and optionally also the current location of all the endoscopes of the pool.

All information can be managed via cloud to allow update of all connected systems and devices. For example, a list of endoscopes with associated ID codes can be sent from the information management system to the network/cloud and can be pushed down from the network/cloud to each endoscope management device.

In the other direction, the endoscope management device can push an event like transportation of the endoscope to the network/cloud and from the network/cloud, the information management system and/or a connected dashboard can be updated.

A planning system can be provided for scheduling planned future uses for the endoscopes of the pool in a set of predetermined locations, e.g. as part of the information system.

When a given endoscope has been placed in a container for transport e.g. to the manual cleaning station and the associated endoscope management device has made this information available in the communication network, the planning system can in particular be configured to send information regarding the next planned use of this particular endoscope to the corresponding endoscope management device, and this additional information comprising e.g. the date, time and location of the next use can also be displayed on the display device.

According to a further aspect of the invention, the object of the invention is solved by an endoscope management method according to the independent method claim, the method using the endoscope management system described above and comprising the following steps:
- detecting an endoscope ID code of a freshly pre-cleaned endoscope by means of the ID reader of one of the endoscope management devices, this endoscope management device thus becoming temporarily associated to the endoscope;
- starting the countdown-timer of the associated endoscope management device indicating or outputting a remaining time until a predetermined time interval ends or a predetermined target time is reached,
- displaying current endoscope information on the display device, and
- making the current endoscope information and preferably also information for identifying the associated EMD available in the communication network;
the current endoscope information comprising:
- a name and/or serial number associated with the detected endoscope ID, and
- the current remaining time as output/indicated by the countdown-timer.

Regarding the advantages of this method and the additional advantages of the preferred embodiments of the method as described below, reference is made to the discussion of the corresponding device or system claims.

There are different possibilities for starting the countdown-timer.

For example, the start of the countdown timer can be automatically triggered by the ID reader of the EMD detecting the endoscope ID, or by the EMD recognizing that the endoscope has been placed inside a transport container, or it can be manually triggered by a user operating a corresponding input element provided on the EMD, e.g. in order to confirm that the endoscope is ready for transport.

In an alternative, it is also possible that any of the above "trigger events", in particular the detection of the endoscope ID by the EMD, triggers the EMD to request the predetermined target time for this endoscope from the information management system and that the start of the countdown timer of the EMD is then triggered by the resulting reception of the predetermined target time by the EMD from the information management system via the network.

The latter alternative is preferred if it is not the end of the manual pre-cleaning step but an earlier event, e.g. the end of the actual diagnostic or therapeutic endoscopic procedure that is supposed to start the relevant countdown until the endoscope has to reach a specific location or stage in the reprocessing procedure.

In this case, it is e.g. possible that the information management system is notified when the endoscope is separated from a videoprocessor in the procedure room after the last procedure, and the resulting target time at which the endoscope should e.g. reach the manual cleaning station is then determined e.g. by the videoprocessor or the information management system. The determined target time is transmitted to the EMD upon request so that the reception of the target time can start the internal countdown by the EMD countdown-timer.

Preferably, the endoscope management method furthermore comprises the following steps:
the information management system providing a list of the ID codes and the associated names and/or serial numbers of all the endoscopes of the pool and making the list available in the communication network (cloud),
pushing down the list from the communication network to all the endoscope management devices of the system.

According to an exemplary embodiment, the method furthermore comprises the following steps:
one of the endoscope management devices of the system recognizing that an event from among the following list of events is taking place or is about to happen or has just happened with respect to one of the endoscopes of the pool:
   - the endoscope is placed inside a container to which the endoscope management device is attached,
   - the endoscope is removed from the container,
   - the container is transported with the endoscope,
   - the container is transported without the endoscope,
pushing information related to the recognized event up to the communication network;
updating the information management system or a dashboard connected to the communication network using the information related to the recognized event.

Finally, the method can also comprise the following steps:
generating a list of future uses for at least one selected endoscope from among the pool, the list comprising a time, date and location of at least one future use of the selected endoscope,
pushing up the list of future uses for the selected endoscope to the communication network,
pushing down the list of future uses from the communication network to the endoscope management device currently associated to the selected endoscope, displaying the list of future uses or information derived from the list of future uses on the display device of the endoscope currently associated to the selected endoscope.

In the following, the present invention is explained in more detail with reference to particular embodiments of the invention as illustrated in the enclosed drawings.
- **Fig. 1**: is a schematic and simplified illustration of an endoscope management device according to an exemplary embodiment of the invention.
- **Fig. 2**: is a schematic and simplified illustration of an endoscope management system according to an exemplary embodiment of the invention, the system comprising several endoscope management devices as shown in Fig. 1.
- **Fig. 3**: is a flow chart illustrating in a simplified manner an endoscope management method according to an embodiment of the invention.
- **Fig. 4**: is a flow chart illustrating in a simplified manner an endoscope management method according to a further embodiment of the invention, and
- **Fig. 5**: is a flow chart illustrating in a simplified manner an endoscope management method according to yet another embodiment of the invention.

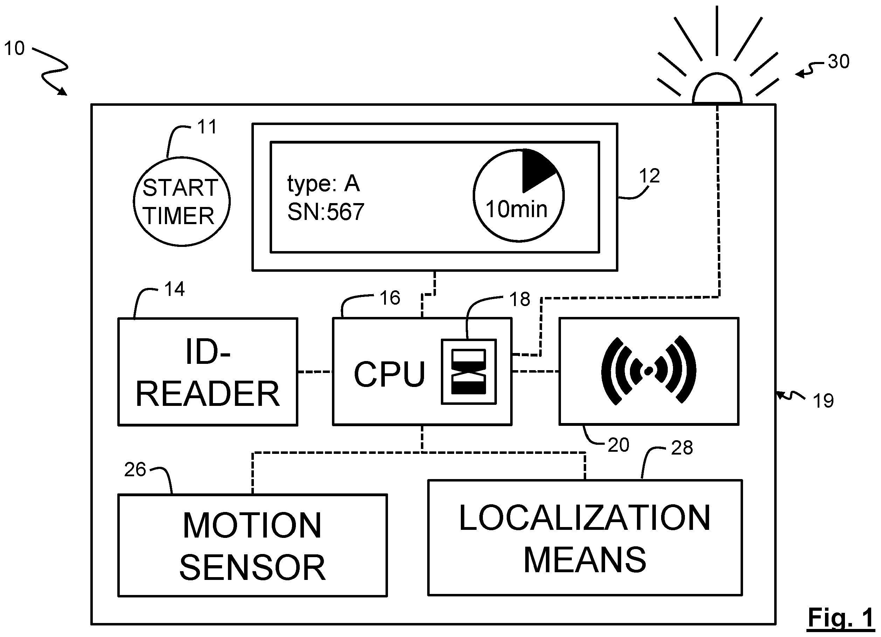

**Fig. 1** illustrates relevant components of an endoscope management device 10 according to an embodiment of the invention in a manner of a block diagram. Exemplary communication links between the different components are indicated as dashed lines. It is noted that the term "component" in this context is to be understood to denote a functional component of the EMD and is not limited to hardware components. A component can thus be implemented by hardware and/or software, and different components can be implemented by the same physical element (e.g. the microprocessor).

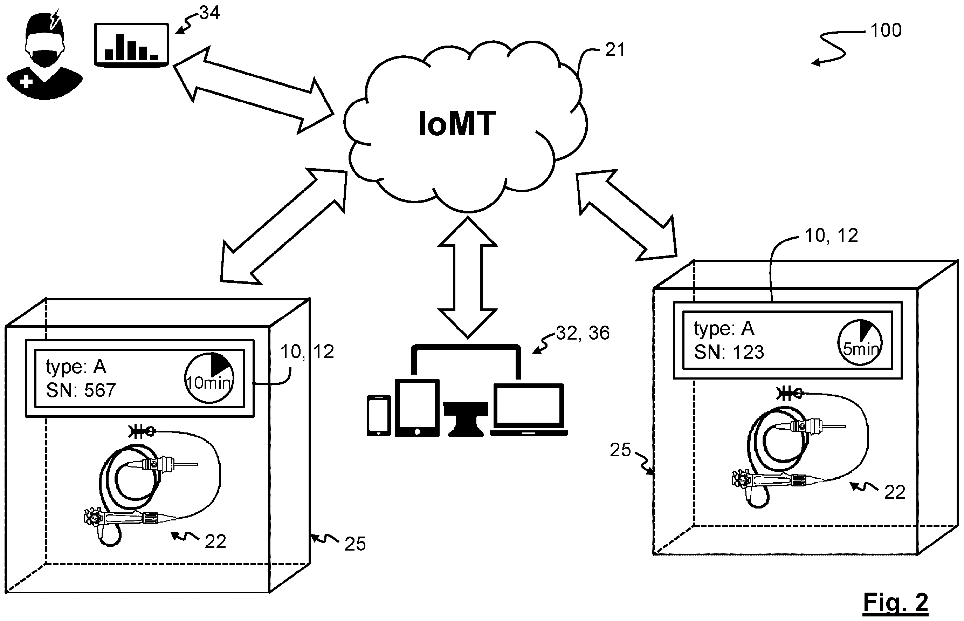

The device 10 comprises a display device 12 e.g. in the form of an e-paper tag, an ID reader 14, e.g. for RFID ID codes, a microprocessor 16 implementing a countdown timer 18 and a communication interface 20 for wirelessly connecting the device 10 to an external communication network such as a hospital WLAN or a cloud network adapted to the specific requirements of healthcare also described as internet of medical things (loMT, cf. **Fig. 2**).

In particular, the countdown timer 18 can be implemented using a real-time clock provided e.g. in the microprocessor (not illustrated).

Preferably, the communication interface 20 uses a low power wireless technology such as Sub-GHz LPWAN, Bluetooth low energy, ANT, ZigBee, RF4CE, NFC, Nike+, Wi-Fi etc.

Optionally, the device 10 can furthermore comprise a motion sensor 26 (e.g. an accelerometer or a gyroscope) and localization means 28 using e.g. LPWAN, Bluetooth or UWB as well as a warning means 30.

All the aforementioned components as well as further components such as a battery and, if desired, additional memory can be accommodated in a compact housing 19 adapted to the hygiene requirements for medical devices. As the size of the housing 19 is mainly determined by the desired screen size for the display device 12, the entire device 10 can also be described as an "loMT enabled endoscope status display device".

After the initial manual pre-clean of the endoscope immediately after its use in the procedure room, the endoscope 22 is scanned by means of the ID-reader 14 of the endoscope management device 10 and is placed in a transport container 25 (cf. Fig. 2). The device 10 is attached to and/or integrated with the container 25, e.g. in a lid portion thereof so that the display device 12 is easily visible.

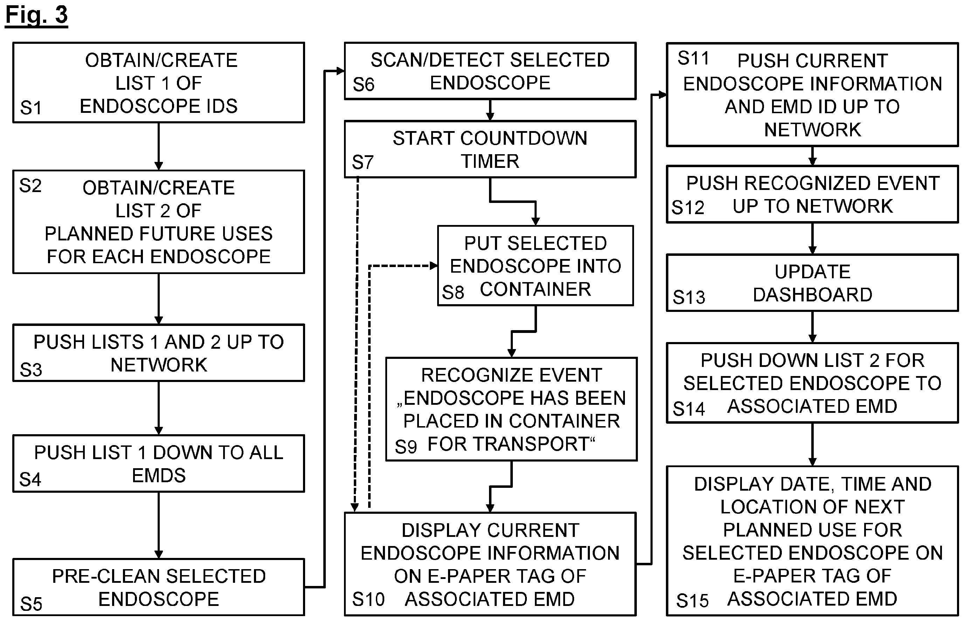

Preferably, scanning the endoscope or any other of the "trigger events" discussed with respect to Figs. 3-5 can start the countdown timer 18, and the name and/or serial number (SN) of the scanned endoscope as well as the remaining time until the endoscope has to arrive at the cleaning station for the next step of the reprocessing procedure are displayed on the display device 12, the remaining time being 10 minutes in the illustrated example of **Fig.1****.**

An optional user input element 11 can be provided on the EMD so that the user can e.g. manually start the countdown-timer (as illustrated) or can manually confirm that a particular event has taken place and should be made available in the network, for example the event that the endoscope has been put in the container and is ready for transport. Although not illustrated, it is possible that also the container 25 has a unique container ID to be detected by the ID reader, in particular if the EMD 10 can be removed from the container 25.

The motion sensor 26 can detect motion patterns indicating that a particular event is taking place or is about to happen or has just happened, e.g. that an endoscope is being placed in the container or is being removed therefrom, or that a transport of the container 25 with or without the endoscope 22 is taking place.

Information related to the detected event can then be made available in the communication network and/or can be displayed on the display device 12.

In addition, the current endoscope information is made available via the communication interface 20 to other users and devices linked to the communication network 21 as illustrated in **Fig. 2****.**

The endoscope management system 100 according to an embodiment of the invention in Fig. 2 comprises a plurality of these endoscope management devices 10, only two of which are shown, as well as an information management system 32 and a dashboard 34 which are all connected to the same communication network 21 as indicated by the double-headed arrows in the Figure.

The information management system 32 can comprise a planning system 36 and can refer to hardware and/or software for managing the current endoscope information made available in the network by all the endoscope management devices 10 of the system 100 as well as further information required for planning, managing and documenting all the diagnostic, therapeutic and reprocessing procedures of all the endoscopes 22 of the pool.

It goes without saying that further devices such as mobile phones or pagers, stationary computers in the procedure rooms, notebooks etc. can be connected to the system 100 in particular in order to assess the current status of any given endoscope 22, the current status comprising information such as the current location, the current reprocessing status and/or information regarding the next planned use of any given endoscope 22 of the pool.

**Fig. 3** shows an exemplary embodiment of an endoscope management method according to the invention. Unless required by logic, the order of the illustrated steps can be changed from the particular order in the illustrations, and additional steps can be performed in between any two steps unless indicated otherwise.

In a first step S1, a first list can be obtained or created e.g. by means of the information management system 32, this first list comprising the unique ID codes of all the endoscopes of a given endoscope pool e.g. in a hospital, together with names and/or serial numbers of the respective endoscopes, optionally together with further relevant information about the respective endoscopes.

In a second step S2, a second list can be obtained or created for each endoscope of the pool, the second list comprising information about at least one planned future use for this endoscope, e.g. the date, time, and location, optionally also the kind of procedure, the name of the patient and of the physician in charge etc.

In a next step S3, these two lists can be pushed up to the network 21. The first list can then be pushed down to all endoscope management devices 10 of the system 100 (step S4).

When, for a selected endoscope 22 the diagnostic/therapeutic procedure is finished, the endoscope is manually pre-cleaned in a step S5, and the selected endoscope is scanned by the ID-reader of an endoscopic management device 10 (short: EMD) which thus detects the unique ID code of the endoscope 22 (step S6). This detection can trigger the start of the countdown timer 18 of the EMD 10 (step S7), the countdown timer indicating the remaining time until the endoscope 22 has to reach the manual cleaning station.

The freshly pre-cleaned endoscope 22 is put into the container 25 to which the EMD 10 is attached (step S8). It is noted that the scanning process (S6) can be performed automatically when the endoscope 22 is inserted in the container 25.

The movement of the container 25 can be measured by means of the motion sensor 26, and from the characteristic motion pattern resulting from the endoscope 22 being inserted into the container, the EMD 10 can recognize this event in a step S9.

The name and serial number of the endoscope as well as the current remaining time before the endoscope 22 has to undergo the next cleaning step at the manual cleaning station are displayed on the display device 12 of the EMD attached to or integrated with the container 25 (step S10). In particular the display of the remaining time is regularly updated, i.e. step S10 is repeated periodically (not illustrated). If the remaining time becomes less than a predetermined threshold, an additional warning signal can optionally be emitted, e.g. a flashing LED underlining the need for rapid processing (not illustrated). It is also possible that step S10 occurs before step S8 as indicated by the dashed arrows.

The current endoscope information (at least remaining time and name and/or serial number of the endoscope) as well as information for identifying the EMD 10 currently associated to this endoscope 22 is pushed up to the network 21 in a step S11, and also the recognized event ("endoscope with serial number xy has been pre-cleaned and is on its way to the cleaning station") can be pushed up to the network 21 (S12).

Using the new information, the dashboard 34 can also be updated (S13). In the illustrated example, this is triggered by the previous "push" steps (S11, S12), but it can also be triggered from other types of data sources such as a reprocessing documentation system.

As it is now known in the network 21 which of the EMDs 10 is currently associated to the selected endoscope 10, the second list comprising information about planned future uses of this endoscope can be pushed down to the associated EMD 10 (S14), and this information can also be shown on the display device 12 of this EMD (S15) so that the healthcare professional in charge of the transport of the endoscope to the cleaning station is informed about when and where this endoscope is needed next.

It is noted that steps such as S5 or S8, which are performed by a user and in which the user does not directly interact with the EMD or the endoscope management system, although shown for illustration in the figures, do not necessarily have to be part of the claimed method.

Steps such as S11 and S12 can happen multiple times, e.g. triggered by a manual step such as S8. When and how often an event is communicated (S12) can depend on different factors. Each communication costs energy so that a balance should be found between keeping the information up-to-date and energy consumption.

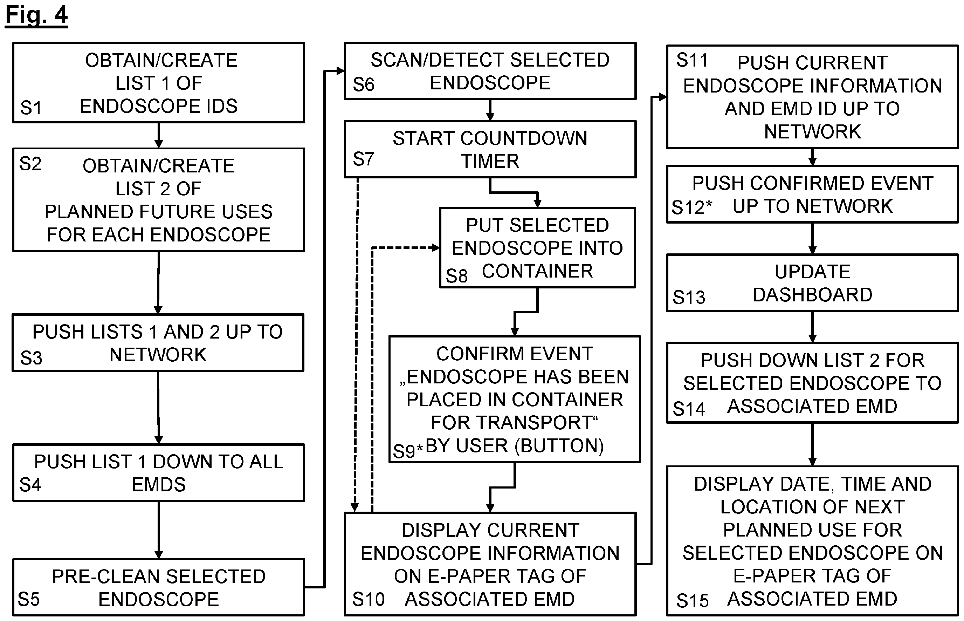

Fig. 4 shows a variant of the method of Fig. 3 which mainly differs from the latter in that the event that the endoscope has been placed in the container for transport is not automatically recognized by the EMD but is manually confirmed by the user (S9*) e.g. by operating a corresponding input element provided on the EMD, and it is this confirmed event that is pushed up to the network in step S12*. In a further variant which is not illustrated here, the manual confirmation in step S9* instead of the detection of the endoscope ID in step S6 could start the countdown-timer (S7).

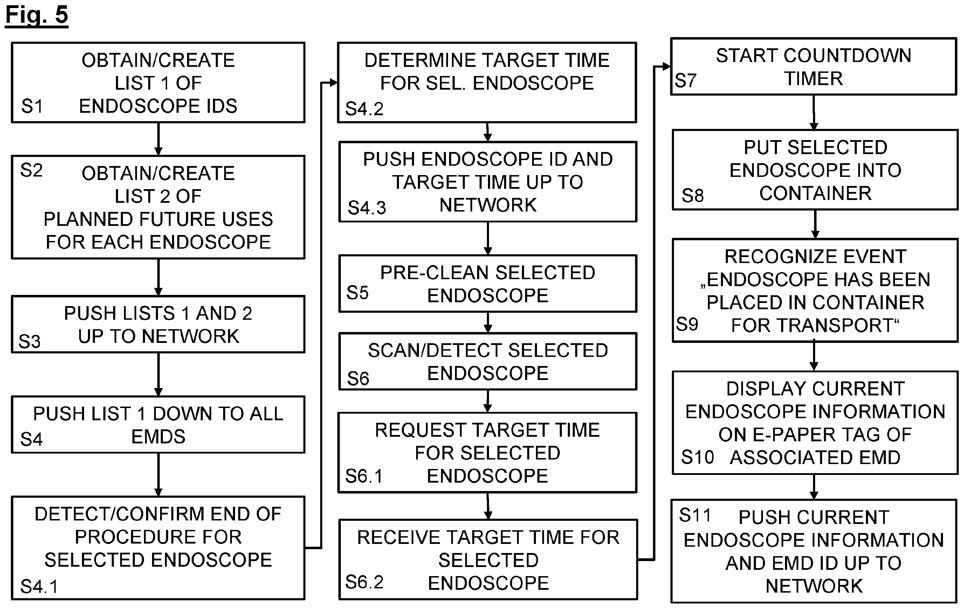

In the method as illustrated in Fig. 5, it is the end of the actual diagnostic or therapeutic endoscopic procedure which determines the target time at which the endoscope has to reach a specific location or stage of the reprocessing procedure, and which is relevant for calculating the "remaining time" to be shown on the display device of the EMD. Therefore, this method comprises a step S4.1 in which the end of the procedure for a selected endoscope is detected, e.g. by a videoprocessor or a connected device recognizing that the endoscope has just been disconnected from the videoprocessor, or confirmed, e.g. by a manual user input. Based on the time at which step S4.1 takes place, the target time for the selected endoscope is determined or calculated in a step S4.2 (e.g. by the videoprocessor or a connected device or by the information management system), and the endoscope ID and the target time for this endoscope are made available in the network in step S4.3.

After or while the selected endoscope is pre-cleaned (S5), the endoscope ID is detected by the ID reader of the EMD (S6), and this detection triggers the EMD to request the target time for the selected endoscope in step S6.1.

When, in response to this request, the EMD receives the target time in step S6.2, this triggers the start of the countdown timer (S7), and for the rest of the illustrated method, reference can be made to the description of Figs. 3 and 4.

### List of Reference Signs

| | |
|---|---|
| endoscope management device | 10 |
| display device | 12 |
| ID reader | 14 |
| microprocessor | 16 |
| countdown-timer | 18 |
| housing | 19 |
| communication interface | 20 |
| communication network | 21 |
| endoscope | 22 |
| container | 25 |
| motion sensor | 26 |
| localization means | 28 |
| warning means | 30 |
| information management system | 32 |
| dashboard | 34 |
| planning system | 36 |

## Claims

1. An endoscope management device (10), the device (10) comprising:
- a display device (12),
- an ID reader (14),
- a microprocessor (16),
- a countdown-timer (18), optionally integrated into the microprocessor (16), and
- a communication interface (20) for wirelessly connecting the endoscope management device (10) to an external communication network (21),
wherein the endoscope management device (10) is configured to:
- detect an endoscope ID code of a freshly pre-cleaned endoscope (22) by means of the ID reader (14);
- start the countdown-timer (18) which outputs a remaining time until a predetermined time interval ends or until a predetermined target time is reached,
- display current endoscope information on the display device (12), and
- make the current endoscope information available in the communication network (21) by means of the communication interface (20);
the current endoscope information comprising:
- a name and/or serial number associated with the detected endoscope ID code, and
- the current remaining time as output by the countdown-timer (18).

2. The endoscope management device (10) according to claim 1, furthermore comprising a housing (19) accommodating the display device (12), wherein the ID reader (14), the microprocessor (16), the countdown-timer (18) and the communication interface (20) are also accommodated in the housing (19).

3. The endoscope management device (10) according to claim 1 or 2, furthermore comprising a motion sensor (26), the microprocessor (16) being configured to collect motion data detected by the motion sensor (26) and to make the detected motion data and/or information derived from the detected motion data available in the communication network (21), wherein the motion sensor (26) preferably is or comprises a gyroscope or an accelerometer.

4. The endoscope management device (10) according to claim 3,
wherein the microprocessor is configured to recognize at least one of and preferably any of the following events, based on the motion data detected by the motion sensor (26):
- the endoscope (22) is placed inside a container (25) to which the endoscope management device (10) is attached,
- the endoscope (22) is removed from the container (25),
- the container (25) is transported with the endoscope (22),
- the container (25) is transported without the endoscope (22),
and wherein the device (10) is configured to make information related to the recognized event available in the communication network (21) and/or to display information related to the recognized event on the display device (12).

5. The endoscope management device (10) according to claim 3 or 4,
wherein the microprocessor (16) is configured to learn to recognize, based on motion data detected by the motion sensor (26), at least one of and preferably all of the following events,
- the endoscope (22) is placed inside a container (25) to which the endoscope management device (10) is attached,
- the endoscope (22) is removed from the container (25),
- the container (25) is transported with the endoscope (22),
- the container (25) is transported without the endoscope (22),
wherein the microprocessor (16) is preferably configured to learn to recognize at least one of and preferably all of the events listed above via tiny machine learning.

6. The endoscope management device (10) according to any of the preceding claims, furthermore comprising localization means (28) for determining a current location of the endoscope management device (10), in particular using Bluetooth or UWB technology,
wherein the endoscope management device (10) is configured to make the determined current location of the device (10) available in the communication network (21).

7. The endoscope management device (10) according to any of the preceding claims,
furthermore comprising acoustic and/or optical warning means (30) such as a flashing LED or a buzzer, the warning means (30) being adapted to emit a warning signal if the current remaining time output by the countdown timer (18) falls below a predetermined threshold.

8. Transport container (25) for an endoscope (22), the container comprising an endoscope management device (10) according to any of the previous claims that is integrated with or attached to the container (25), preferably removably or exchangeable attached to the container (25).

9. An endoscope management system (100) for a pool of endoscopes (22), each endoscope (22) of the pool having a unique ID code, the system (100) comprising:
a plurality of endoscope management devices (10) according to any of claims 1 to 7 and/or a plurality of transport containers (25) according to claim 8, the endoscope management devices (10) all being connected or connectable to the same communication network (21);
the endoscope management system (100) furthermore comprising an information management system (32) connected or connectable to the communication network (21), the information management system (32) being configured to manage the current endoscope information made available by all the endoscope management devices (10).

10. The endoscope management system (100) according to claim 9,
the system (100) furthermore comprising a dashboard (34) configured to display the current endoscope information and optionally the current location of all the endoscopes (22) of the pool.

11. The endoscope management system (100), according to claim 9 or 10, furthermore comprising a planning system (36) for scheduling planned future uses of the plurality of endoscopes in a set of predetermined locations.

12. Endoscope management method using the endoscope management system (100) of any of the previous system claims, the method comprising the following steps:
- detecting (S6) an endoscope ID code of a freshly pre-cleaned endoscope (22) by means of the ID reader (14) of one of the endoscope management devices (10), this endoscope management device (10) thus becoming temporarily associated to the endoscope (22);
- starting (S7) the countdown-timer (18) of the associated endoscope management device (10) indicating or outputting a remaining time until a predetermined time interval ends or a predetermined target time is reached,
- displaying (S10) current endoscope information on the display device (12), and
- making (S11) the current endoscope information and preferably also information for identifying the associated endoscope management device (10) available in the communication network (21);
the current endoscope information comprising:
- a name and/or serial number associated with the detected endoscope ID, and
- the current remaining time as indicated or output by the countdown-timer (18).

13. Endoscope management method according to claim 12, furthermore comprising the following steps:
the information management system (32) providing (S1) a list of the ID codes and the associated names and/or serial numbers of all the endoscopes (22) of the pool and making (S3) the list available in the communication network (21),
pushing down (S4) the list from the communication network (21) to all the endoscope management devices (10) of the system (100).

14. Endoscope management method according to claim 12 or 13, furthermore comprising the following steps:
one of the endoscope management devices (10) of the system (100) recognizing (S9) that an event from among the following list of the events is taking place or is about to happen or has just happened with respect to one of the endoscopes (22) of the pool:
- the endoscope (22) is placed inside a container (25) to which the endoscope management device (10) is attached,
- the endoscope (22) is removed from the container (25),
- the container (25) is transported with the endoscope (22),
- the container (25) is transported without the endoscope (22),
pushing (S12) information related to the recognized event up to the communication network (21);
updating (S13) the information management system (32) or a dashboard (34) connected to the communication network (21) using the information related to the recognized event.

15. Endoscope management method according to any of the preceding method claims, the method furthermore comprising the following steps:
generating (S2) a list of future uses for at least one selected endoscope (22) from among the pool, the list comprising a time, date and location of at least one future use of the selected endoscope (22),
pushing up (S3) the list of future uses for the selected endoscope (22) to the communication network (21),
pushing down (S14) the list of future uses from the communication network (21) to the endoscope management device (10) currently associated to the selected endoscope (22),
displaying (S15) the list of future uses or information derived from the list of future uses on the display device (12) of the endoscope management device (10) currently associated to the selected endoscope (22).

16. Endoscope management method according to any of the preceding method claims, wherein the step of starting (S7) the countdown timer (18) is triggered by one of the three following trigger events:
- the ID reader (14) of the endoscope management device (10) detecting (S6) the endoscope ID code of the freshly cleaned endoscope (22),
- the endoscope management device (10) recognizing (S9) that the endoscope (22) has been placed inside a transport container (25),
- a user operating (S9*) an input element (11) provided on the endoscope management device (10),
or wherein one of the above three trigger events (S6, S9, S9*) triggers the endoscope management device (10) to request (S6.1) the predetermined target time for the endoscope (22) from the information management system (32), and wherein the endoscope management device (10) receiving (S6.2) the requested target time from the information management system (32) triggers the start (S7) of the countdown timer (18).
